# EUROPÄISCHE PATENTANMELDUNG

(11) **EP 2 030 560 A2**
(43) Veröffentlichungstag der Anmeldung: **04.03.2009**
(21) Anmeldenummer: 08075713.1
(22) Anmeldetag: 20.08.2008
(51) Int. Cl.: A61B 3/00, A61B 19/00, G02B 21/00

(54) **Sekundäire Lichtquelle**

(30) Priorität: 28.08.2007 DE 102007041439
(71) Anmelder: Carl Zeiss Surgical GmbH, 73447 Oberkochen (DE)
(72) Erfinder: Nahm, Werner, Dr., 74426 Bühlerzell (DE); Matz, Holger, Dr., 73485 Unterschneidheim (DE); Bausewein, Markus, 73431 Aalen (DE); Reimer, Peter, 73479 Ellwangen (DE)
(74) Vertreter: Theobald, Andreas

(57) **Zusammenfassung**

Es wird eine sekundäre Lichtquelle einer schmalbandiges Licht emittierenden schmalbandigen Lichtquelle (11) als einer primären Lichtquelle, einem Lichtleiter (5) mit einem proximalen und einem distalen Ende (9), einer am proximalen Ende (7) des Lichtleiters (5) angeordneten Einkopplungsvorrichtung (13) zum Einkoppeln des schmalbandigen Lichts in den Lichtleiter (5) und einem an oder vor dem distalen Ende (9) des Lichtleiters (5) vorhandenen Leuchtstoffbereich (19), der mit einem Konverter-Leuchtstoff versehen ist, zur Verfügung gestellt. Der Konverter-Leuchtstoff des Leuchtstoffbereiches (19) ist derart im Bezug auf das von der schmalbandigen Lichtquelle (11) emittierte schmalbandige Licht gewählt, dass die Wellenlänge wenigstens eines Teils des schmalbandigen Lichtes von ihm vergrößert.

## Beschreibung

Die vorliegende Erfindung betrifft eine sekundäre Lichtquelle sowie eine medizinische Illuminationsvorrichtung und eine optische Kohärenztomographie-Vorrichtung (OCT).

In der Medizintechnik werden häufig so genannte Endoilluminatoren eingesetzt, um bspw. bei Eingriffen am Auge dieses von Innen zu beleuchten, was insbesondere bei Eingriffen am hinteren Augenabschnitt von Bedeutung ist. Auch bei anderen mikrochirurgischen oder endoskopischen Eingriffen in Körperhöhlen können solche Endoilluminatoren zum Einsatz kommen.

Standardmäßig wird dabei das Licht von Halogen-, Xenon-, Metallhalogenidlampen oder von hochleistungs-LEDs in Lichtleitfasern eingekoppelt und über entsprechende Handstücke, so genannte Applikatoren, in die Körperhöhlen eingebracht. Dabei sollen die Lichtquellen möglichst klein sein, um das Licht möglichst effektiv in den Lichtleiter einkoppeln. Am distalen Ende des Lichtleiters sollte das Licht möglichst gleichförmig und je nach Anwendung gerichtet oder diffus in den kompletten Raum abgestrahlt werden. Manchmal ist es auch sinnvoll, die Farbe bzw. die Farbtemperatur des abgestrahlten Lichtes zu variieren. Bei klassischen Lichtquellen wie Halogen-, Xenon- und Metallhalogenidlampen kann dies über Filter erfolgen. Bei LEDs als Lichtquellen müssen LEDs unterschiedlicher Farbe eingesetzt werden, was die Einkopplung des Lichtes in die Lichtleitfasern erschwert.

Die bisher gebräuchlichen Endoilluminatoren basierend auf Halogen-, Xenon- oder Metallhalogenidlampen können zwar über Filter farblich variiert werden, weisen aber eine schlechte Effizienz auf und erzeugen relativ hohe Verluste in Form von Abwärme. Da zudem die emittierende Fläche relativ groß ist, lässt sich das Licht auch nur schwer bzw. uneffektiv in dünne Lichtleiter einkoppeln.

Endoilluminatoren basierend auf hochleistungs-LEDs sind in der Farbe nur variierbar, wenn verschiedenfarbige LEDs verwendet werden. Auch dies vergrößert wieder die abstrahlende Fläche und erschwert damit das Einkoppel in dünne Lichtleiter. Zudem ist die Abstrahlung am distalen Lichtleiterende oft inhomogen und stark gerichtet. Um hier eine homogene Abstrahlung, die eventuell sogar in den nahezu kompletten Raumwinkel erfolgen soll, zu bekommen, müssen besondere Maßnahmen wie etwa Lichtmischer oder Diffusoren eingesetzt werden.

Ein Endoilluminator zum Projizieren von Beleuchtungslicht in das Innere eines Augapfels ist beispielsweise in US 2004/0249424 A1 beschrieben. Er umfasst eine starre Hohlnadel, mit der der Augapfel durchstochen werden kann. Durch die Nadel erstreckt sich eine optische Faser, deren distales Ende eine sekundäre Lichtquelle darstellt, die im Augapfel angeordnet werden kann. Am proximalen Ende der Faser ist ein Verbindungsstück vorhanden, mit dem die optische Faser an eine konventionelle Lampe als primäre Lichtquelle angeschlossen werden kann. Als Lichtquellen finden derzeit üblicherweise Halogen- oder Xenonlampen Anwendung. Bei derartigen Lichtquellen ist für eine ausreichende Abfuhr der erzeugten Wärme zu sorgen.

Aus US 2004/0090796 A1 ist zudem ein Endoilluminator mit einer LED als primärer Lichtquelle beschrieben. LEDs besitzen im Vergleich zu Halogen- oder Xenonlampen eine längere Lebensdauer und entwickeln weniger Wärme. Das Einkoppeln von LED-Licht in die optische Faser bereitet jedoch wie bereits erwähnt oftmals Probleme hinsichtlich der Effizienz und der Lichtleistung, was die Intensität des Lichtes am Ausgangsende der Faser, also die Intensität der sekundären Lichtquelle, negativ beeinflusst. Auch die Abstrahlung am distalen Ende des Lichtleiters ist wie weiter oben erwähnt nicht optimal und erfordert daher ggf. weitere Maßnahmen.

JP2006261077 beschreibt eine Lichtquelle mit einer ultraviolettes Licht, violettes Licht, blaues Licht oder weißes Licht emittierenden LED und einer optischen Faser, auf deren Eingangsende eine fluoreszierende Beschichtung aus einem Konverterleuchtstoff aufgebracht ist. Mittels des Leuchtstoffes findet eine Farbkonversation des Lichtes der LED statt.

Aufgabe der vorliegenden Erfindung ist es daher, eine vorteilhafte sekundäre Lichtquelle zur Verfügung zu stellen, die sich insbesondere in einer medizinischen Illuminationsvorrichtung oder in einem OCT einsetzen lässt.

Diese Aufgabe wird durch eine sekundäre Lichtquelle nach Anspruch 1 gelöst. Die abhängigen Ansprüche enthalten vorteilhafte Ausgestaltungen der Erfindung.

Eine erfindungsgemäße sekundäre Lichtquelle umfasst eine schmalbandiges Licht emittierende schmalbandige Lichtquelle als primäre Strahlungsquelle, einen Lichtleiter mit einem proximalen und einem distalen Ende und eine am proximalen Ende des Lichtleiters angeordnete Einkopplungsvorrichtung zum Einkoppeln des schmalbandigen Lichts in den Lichtleiter. An dem distalen Ende oder dem distalen Ende des Lichtleiters in Richtung auf das proximale Ende vorgelagert ist ein mit einem Konverter-Leuchtstoff versehener Leuchtstoffbereich vorhanden, der volumig oder flächig ausgebildet sein kann. Unter dem Begriff "schmalbandiges Licht" soll hierbei auch knapp außerhalb des spektralen Wahrnehmungsvermögens des menschlichen Auges liegende Strahlung zu verstehen sein, insbesondere Licht im nahen Ultraviolett. Der Konverter-Leuchtstoff des Leuchtstoffbereiches ist derart mit Bezug auf das von der schmalbandigen Lichtquelle emittierte schmalbandige Licht gewählt, dass die Wellenlänge wenigstens eines Teils des schmalbandigen Lichtes von ihm vergrößert wird. Insbesondere kann er so gewählt sein, dass am distalen Ende des Lichtleiters weißes oder breitbandiges Licht austritt. Als Lichtleiter eignet sich insbesondere ein Glasfaserstab oder eine flexible Lichtleitfaser. Als schmalbandige Lichtquellen können bspw. LEDs. Laser oder Laserdioden zur Anwendung kommen.

Mit der erfindungsgemäßen sekundären Lichtquelle wird davon abgerückt, eine Weißlichtquelle als primäre Strahlungsquelle für die in den Lichtleiter einzukoppelnde Strahlung zu verwenden. Stattdessen findet eine schmalbandige Lichtquelle Verwendung, deren schmalbandiges Licht in den Lichtleiter eingekoppelt wird. Erst im Lichtleiter und insbesondere kurz vor dem oder beim Austritt aus dem Lichtleiter wird das schmalbandige Licht in weißes Licht oder breitbandiges Licht umgewandelt, oder es erfolgt eine Farbkonversion. Dazu wandelt der Konverter-Leuchtstoff wenigstens einen Anteil des schmalbandigen Licht in Licht mit einer längeren Wellenlänge als die des ursprünglichen schmalbandigen Lichts um.

Wenn als schmalbandiges Licht sichtbares Licht in den Lichtleiter eingekoppelt wird, kann ein Teil des eingekoppelten Lichtes mittels des Konverter-Leuchtstoffes in Licht mit einer längeren Wellenlänge umgewandelt werden, so dass die Überlagerung des umgewandelten Lichtes mit dem verbleibenden Rest des ursprünglich eingekoppelten, nicht umgewandelten Lichtes zu weißem Licht führt. Beispielsweise ist es möglich, eine blaues Licht emittierende schmalbandige Lichtquelle zu verwenden. Der Konverter-Leuchtstoff kann dann so gewählt sein, dass er einen Teil des blauen Lichtes in gelbes Licht umwandelt, so dass die Überlagerung des gelben Lichtes mit dem verbleibenden blauen Licht weißes Licht ergibt. Wenn dagegen beispielsweise UV-Strahlung in den Lichtleiter eingekoppelt wird, ist es möglich, durch Verwendung eines Konverter-Leuchtstoffes die UV-Strahlung vollständig in Licht im sichtbaren Spektralbereich umzuwandeln. Zudem ist es möglich, durch Verwendung eines Konverter-Leuchtstoffes, der ein Gemisch aus verschiedenen Leuchtstoffen ist, die UV-Strahlung vollständig in Licht mit wenigstens zwei, in der Summe zu weißem Licht führenden Wellenlängenverteilungen oder in Licht mit einer breitbandigen Wellenlängenverteilung umzuwandeln. Das Verwenden eines Gemisches als Konverter-Leuchtstoff zur Realisierung einer weiß oder breitbandig emittierenden sekundären Lichtquelle ist aber nicht nur bei UV-Strahlung, sondern auch bei Verwendung einer sichtbares Licht emittierenden schmalbandigen Lichtquelle möglich.

Ein weiterer, sich aus der Erfindung ergebender Vorteil ist, dass der Konverter-Leuchtstoff, der in der Regel auf Fluoreszenz beruht, das Fluoreszenzlicht gleichmäßig in alle Raumrichtungen abstrahlt, so dass man eine sehr homogene Lichtverteilung am Ausgangsende des Lichtleiters erhält, die über die räumliche Verteilung des Konverter-Leuchtstoffes noch zusätzlich beeinflusst werden kann. Bspw. ist es denkbar, den Konverter-Leuchtstoff in Form von kleinen Kugeln in das distale Ende des Lichtleiters einzubringen.

Bei Verwendung der sekundären Lichtquelle in Endoilluminatoren können diese auch für Fluoreszenzanwendungen oder für photodynamische Therapien eingesetzt werden, wenn Konverter-Leuchtstoffe mit entsprechenden Emissionsspektren zum Einsatz kommen.

Insbesondere durch die Verwendung eines Lasers, einer Laserdiode oder einer einzigen LED als primärer Lichtquelle kann im Vergleich zu der Verwendung von Lampen oder weißen LEDs als Lichtquellen in der erfindungsgemäßen sekundären Lichtquelle das Einkoppeln des Lichtes in den Lichtleiter hinsichtlich der Effizienz und der Lichtleistung optimiert werden. Eine einzelne LED weist bspw. gegenüber einer Anordnung aus LEDs mit unterschiedlichen Farben eine geringere emittierende Fläche auf, was das Einkoppeln vereinfacht. Auch gegenüber einer weißes Licht emittierenden LED ist das Licht einer schmalbandiges Licht emittierenden LED leichter einzukoppeln, da eine weißes Licht emittierenden LED mit einem Konverter-Leichtstoff beschichtet ist, der zu einer Abstrahlung in einen großen Raumwinkel führt, was die Einkopplung verkompliziert. Die Verwendung eines Lasers, einer Laserdiode oder einer einzigen LED als primärer Lichtquelle verringert daher die beim Einkoppeln auftretenden Verluste und erhöht damit die Effizienz der sekundären Lichtquelle.

Der Konverter-Leuchtstoff kann als Dotierung in den Leuchtstoffbereich eingebracht sein oder, wenn sich der Leuchtstoffbereich am distalen Ende des Lichtleiters befindet, als Beschichtung auf das distale Ende des Lichtleiters aufgebracht sein. Mit dem Dotieren lassen sich insbesondere volumige Leuchtstoffbereiche einfach herstellen. Das Herstellen flächiger Leuchtstoffbereiche kann hingegen durch Beschichten erfolgen. Aber auch das Dotieren lediglich einer dünnen Oberflächenschicht am distalen Ende führt zu einem im Wesentlichen flächigen Leuchtstoffbereich.

Für die Verwendung mit einer blaues Licht emittierenden schmalbandigen Lichtquelle eignen sich insbesondere die Konverter-Leuchtstoffe aus der Gruppe: YAG:Ce, ThAG:Ce, SrGa₂S₄:Eu, Ca₈EuMnMg(SiO₄)₄C₁₂, CaS:Eu und SrS:Eu sowie Gemische aus diesen Stoffen. Diese Stoffe sind beispielsweise aus TW 245433 B bekannt. Während ThAG:Ce wie YAG:Ce das blaue Licht in gelbes Licht umwandelt, wandeln SrGa₂S₄:Eu und Ca₈EuMnMg(SiO₄)₄C₁₂ blaues Licht in grünes Licht um und CaS:Eu sowie SrS:Eu in rotes Licht. Insbesondere durch eine geeignete Mischung der genannten Leuchtstoffe lässt sich so beispielsweise die Charakteristik des aus dem Lichtleiter austretenden Lichtes gezielt einstellen. Für die Anwendung der sekundären Lichtquelle ist dabei insbesondere für den Fall auf die Bioverträglichkeit des Konverter-Leuchtstoffes zu achten, dass er als Beschichtung auf das distale Ende aufgebracht ist.

In einer vorteilhaften Weiterbildung der sekundären Lichtquelle schließt sich an den Leuchtstoffbereich in Richtung auf das proximale Ende des Lichtleiters eine dichroite Verspiegelung an. Diese ist so ausgebildet, dass sie sich in Richtung auf das proximale Ende ausbreitendes Licht reflektiert. Dadurch lässt sich vermeiden, dass im Konverter-Leuchtstoff entstehendes Licht in Richtung auf das proximale Ende des Lichtleiters geleitet wird, statt vom distalen Ende emittiert zu werden. Die Intensität des konvertierten Lichtes am distalen Ende lässt sich daher mit Hilfe der dichroiten Verspiegelung im Vergleich zu Lichtleiter ohne eine derartige Verspiegelung erhöhen, was die Effizienz der sekundären Lichtquelle weiter erhöht. Insbesondere kann die Verspiegelung auch so ausgebildet sein, dass sie nur für das konvertierte Licht hoch reflektiv, für das ursprüngliche schmalbandige Licht jedoch durchlässig ist.

Weiterhin ist es möglich, am Ausgang des distalen Endes des Lichtleiters eine teildurchlässige Verspiegelung anzuordnen, die für phototoxische Wellenlängenbestandteile des ursprünglichen schmalbandigen Lichts hoch reflektiv ist, für die nicht phototoxischen Bestandteile sowie das konvertierte Licht jedoch durchlässig ist. Auf diese Weise können die phototoxischen Bestandteile bspw. bei Verwendung der sekundären Lichtquelle in einem Endoilluminator vom Gewebe fern gehalten werden. Gleichzeitig wird der reflektierte Anteil des Lichtes wieder dem Konverter-Leuchtstoff zugeführt, was die Effizienz der Konversion erhöht. Insbesondere im Zusammenwirken mit der oben erwähnten, die Lichtausbreitung in Richtung auf das proximale Ende des Lichtleiters verhindernden Verspiegelung kann damit eine nahezu hundertprozentige Konversion erreicht werden.

Besonders vorteilhaft ist es, wenn als Lichtleiter eine so genannte Monomode-Faser Verwendung findet. Monomode-Fasern sind sehr dünne optische Fasern, die lediglich die Ausbreitung einer einzigen Schwingungsmode der elektromagnetischen Strahlung erlauben. Derartige Monomode-Fasern lassen sich mit sehr kleinen Faserdurchmessern realisieren, so dass beispielsweise bei Endoilluminatoren zum Beleuchten des Inneren des Augapfels lediglich eine sehr kleine Öffnung im Augapfel vorhanden sein muss. Aufgrund des geringen Faserdurchmessers im Bereich von 8-10 µm ist das Einkoppeln von weißem LED-Licht oder von Licht thermischer Strahler wie etwa Lampen in Monomode-Fasern schwierig. Die Ursache hierfür liegt in dem großen Raumwinkel, in den die weiße LED ebenso wie eine Lampe emittiert. Insbesondere Laser, aber auch schmalbandige LEDs emittieren dagegen in einen kleineren Raumwinkel, da die Strahlung gebündelt ist. Dies ist insbesondere bei einem Laser als primärer Lichtquelle der Fall, so dass das Laserlicht beispielsweise mittels einer oder mehrere Linsen gut in Lichtleitfasern und insbesondere in Monomode-Fasern einzukoppeln ist. Durch den Leuchtstoffbereich kann dann das schmalbandige Licht in weißes oder breitbandiges Licht umgewandelt werden. So kann am Austrittsende der Monomode-Faser weißes oder breitbandiges Licht mit einer Intensität emittiert werden, die bei Verwendung einer weißen LED oder einer Argon- bzw. Xenonlampe nicht erreichbar wäre.

Die erfindungsgemäße sekundäre Lichtquelle kann beispielsweise vorteilhaft als Lichtquelle in einem Endoilluminator oder in anderen medizinischen Illuminationsvorrichtungen, etwa im Bereich der Endoskopie, Verwendung finden. Insbesondere eignet sich die erfindungsgemäße sekundäre Lichtquelle auch zur Verwendung in einem OCT. Der grundsätzliche Aufbau eines OCT ist beispielsweise in DE 199 29 406 beschrieben.

Weitere Merkmale, Eigenschaften und Vorteile der vorliegenden Erfindung ergeben sich aus der nachfolgenden Beschreibung von Ausführungsbeispielen unter Bezugnahme auf die beiliegenden Figuren.
- Figur 1: zeigt einen Endoilluminator mit einer erfindungsgemäßen sekundären Lichtquelle.
- Figur 2: zeigt ein Detail aus Figur 1.
- Figur 3: zeigt eine alternative Ausgestaltung der sekundären Lichtquelle im Detail.
- Figur 4: zeigt eine Weiterentwicklung der sekundären Lichtquelle im Detail.
- Figur 5: zeigt eine Abwandlung der in Figur 4 dargestellten Weiter- entwicklung im Detail.
- Figur 6: zeigt einen alternativen Endoilluminator mit einer erfindungsgemäßen sekundären Lichtquelle.
- Figur 7: zeigt ein OCT mit einer erfindungsgemäßen sekundären Lichtquelle.
- Figur 8: zeigt die sekundäre Lichtquelle des OCT aus Figur 6.
- Figur 9: zeigt ein Spektrum des von der sekundären Lichtquelle emittierten Lichts.

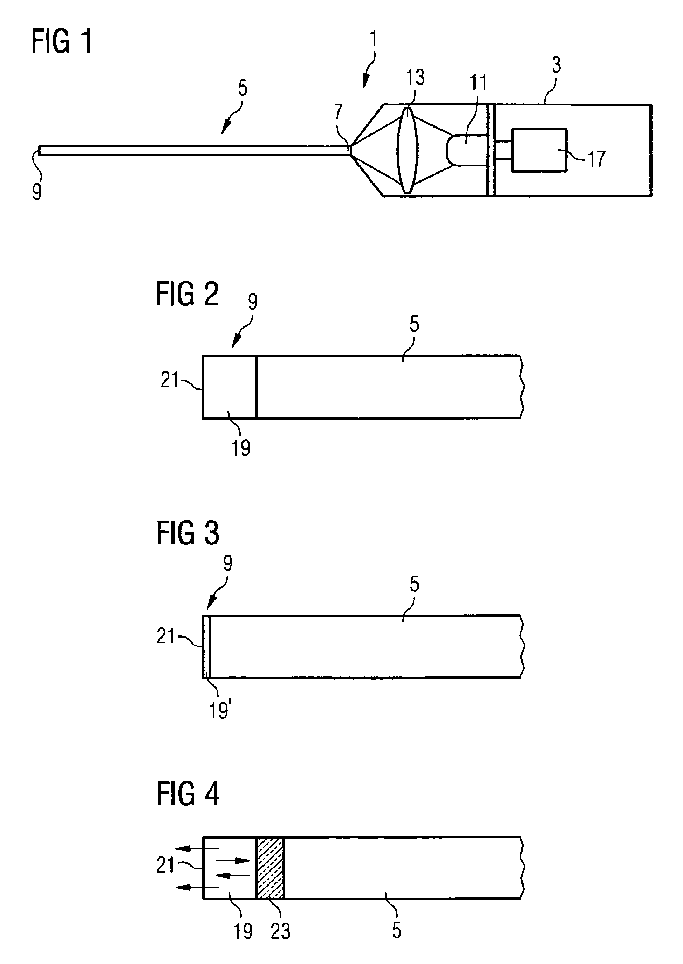

Figur 1 zeigt als ein Ausführungsbeispiel für eine medizinische Illuminationsvorrichtung mit einer erfindungsgemäßen sekundären Lichtquelle einen Endoilluminator 1 in einer stark schematisierten Darstellung. Der Endoilluminator 1 umfasst einen Handgriff 3 und einen Glasfaserstab 5. Der Glasfaserstab 5 dient als Lichtleitfaser mit einem proximalen Ende 7, in das Licht einer im Inneren des Handgriffs 3 angeordneten primären Lichtquelle 11 eingekoppelt wird, und einem distalen Ende 9, zu dem das eingekoppelte Licht geleitet wird, und aus dem es austritt.

Im vorliegenden Ausführungsbeispiel ist der Glasfaserstab als Monomode-Faser ausgebildet. Der Durchmesser des Glasfaserstabes ist daher sehr gering (8-10 µm), so dass er durch lediglich kleine Öffnungen in den Körper eingeführt werden kann, wodurch sich Traumata minimieren lassen.

Obwohl in den Figuren nicht explizit dargestellt, ist der Glasfaserstab 5 von einem Mantel umgeben, der ebenfalls dünn sein kann. Dieser kann außerdem von einer Schützhülle umgeben sein, die insbesondere auch sterilisierbar sein kann.

Der Glasfaserstab 5 kann, wie in Figur 1 dargestellt, fest mit dem Handgriff 3 verbunden sein. Er kann alternativ jedoch auch an seinem proximalen Ende 7 eine optische Steckverbindung aufweisen, die mit einer entsprechenden optischen Steckverbindung am Handgriff 3 zum Verbinden des Glasfaserstabes mit dem Handgriff 3 zusammenwirken kann. Eine derartige Steckverbindung würde ein einfaches Auswechseln des Glasfaserstabs 5 ermöglichen.

Im Handgriff 3 ist zudem eine Elektronik 17 zum geeigneten Versorgen der primären Lichtquelle 11 mit Spannung vorhanden. Darüber hinaus kann auch eine Energiequelle, beispielsweise ein Akku, in den Handgriff 3 integriert sein. Alternativ ist es möglich, den Handgriff 3 mittels einer nicht dargestellten elektrischen Leitung an eine externe Energievorsorgung, beispielsweise ein öffentliches Leitungsnetz, anzuschließen.

Die primäre Lichtquelle ist im vorliegenden Ausführungsbeispiel eine Laserdiode 11. Das von der Laserdiode 11 ausgestrahlte Licht wird von einer Einkopplungsvorrichtung, die in Figur 1 durch eine Linse 13 angedeutet ist, in das proximale Ende 7 des Glasfaserstabs 5 eingekoppelt. Obwohl durch eine Linse 13 angedeutet, kann die Einkopplungsvorrichtung zusätzlich zu Linsen oder alternativ zu Linsen auch Spiegel und andere optische Elemente umfassen. Im dargestellten Endoilluminator 1 kommt eine Laserdiode 11 zur Anwendung, die blaues Laserlicht emittiert. Das blaue Laserlicht wird mittels der Einkopplungsvorrichtung 13 in das proximale Ende 7 des Glasfaserstabes 5 eingekoppelt, von wo aus es zum distalen Ende 9 geleitet wird.

Figur 2 zeigt eine Detaildarstellung des distalen Endes 9 des Glasfaserstabes 5. Am distalen Ende 9 befindet sich ein Leuchtstoffbereich 19, in dem das Glasfasermaterial mit YAG:Ce dotiert ist. YAG:Ce bildet einen Konverter-Leuchtstoff, der einen Teil des am distalen Ende 9 ankommenden blauen Laserlichtes in gelbes Licht umwandelt. Die YAG:Ce-Konzentration im Leuchtstoffbereich 19 ist dabei so gewählt, dass der Anteil an zu gelbem Licht konvertiertem blauem Licht gerade so hoch ist, dass das Gemisch aus blauem und gelbem Licht beim Austritt aus dem distalen Ende 9 weitgehend weiß erscheint. Je größer die axiale Ausdehnung des Leuchtstoffbereiches 19 im distalen Ende des Glasfaserstabes ist, desto geringer braucht die Konzentration des YAG:Ce zu sein.

Alternativ zum YAG:Ce kann der Leuchtstoffbereich 19 als Konverter-Leuchtstoff ThAG:Ce enthalten. Falls die sekundäre Lichtquelle besonders breitbandig emittieren soll, kann der Konvertoer-Leuchtstoff ein Gemisch aus wenigstens zwei der folgenden Stoffe enthalten: YAG:Ce, ThAG:Ce, SrGa₂S₄:Eu, Ca₈EuMnMg(SiO₄)₄C₁₂, CaS:Eu oder SrS:Eu.

Das Dotieren kann beispielsweise erfolgen, indem der entsprechende Stoff auf die Oberfläche des Leuchtstoffbereiches 19 aufgebracht wird und diese Anschließend einer Wärmebehandlung unterzogen wird, bei der der Stoff in das Glasfasermaterial diffundiert. Alternativ kann das Einbringen des Dotierstoffes auch mittels Implantation erfolgen. Die Implantationen kann hierbei ggf. durch eine anschließende Wärmebehandlung unterstützt werden, bei der eine Diffusion des implantieren Dotierstoffes erfolgt.

Eine alternative Ausgestaltung des Leuchtstoffbereiches ist in Figur 3 dargestellt. In dieser Variante des distalen Endes 9 des Glasfaserstabes 5 ist der Leuchtstoffbereich 19' nicht wie in Figur 2 volumig ausgebildet, sondern flächig. Der flächige Leuchtstoffbereich 19' ist durch eine Beschichtung gebildet, die auf das Austrittsende 21 des Glasfaserstabes 5 aufgebracht ist. Die Beschichtung kann mit allen geeigneten Beschichtungsverfahren, etwa mittels chemischer oder physikalischer Abscheidung aus der Gasphase (CVD bzw. PVD), aufgebracht werden. Die Beschichtung umfasst im vorliegenden Ausführungsbeispiel YAG:Ce. Sie kann jedoch grundsätzlich dieselben Stoffe umfassen, wie sie mit Bezug auf den volumigen Leuchtstoffbereich 19 aus Figur 2 beschrieben worden sind.

Obwohl der flächige Leuchtstoffbereich 19' im vorliegenden Ausführungsbeispiel im Form einer Beschichtung realisiert ist, kann er grundsätzlich auch durch eine flache, d.h. oberflächennahe Dotierung erzeugt werden.

Eine Weiterentwicklung der sekundären Lichtquelle, deren distales Ende in Figur 2 dargestellt ist, ist in Figur 4 gezeigt. Diese Figur zeigt wie Figur 2 das distale Ende 9 des Glasfaserstabes 5 mit dem dort vorhandenen volumigen Leuchtstoffbereich 19. Im Unterschied zur in Figur 2 dargestellten Variante schließt sich in der in Figur 4 dargestellten Variante an den Leuchtstoffbereich 19 in Richtung auf das proximale Ende 7 des Glasfaserstabes 5 eine dichroite Verspiegelung 23 an. Diese ist so ausgestaltet, dass sie das vom proximalen Ende kommende blaue Licht ungestört hindurchtreten lässt. Das im Leuchtstoffbereich 19 entstehende gelbe Licht wird hingegen reflektiert. Durch die dichroite Verspiegelung wird also gelbes Licht, das sich vom Leuchtstoffbereich in Richtung auf das proximale Ende des Glasfaserstabes 5 ausbreitet, in Richtung auf das distale Ende 9 des Glasfaserstabes 5 reflektiert. Auf diese Weise wird erreicht, dass auch gelbes Licht, das sich nach der Emission in Richtung auf das proximale Ende 7 ausbreitet, zur Beleuchtung beiträgt, so dass die Ausbeute am weißen Licht am distalen Ende 9 des Glasfaserstabes 5 optimiert ist und die sekundäre Lichtquelle eine optimale Intensität besitzt.

Selbstverständlich kann die dichroite Verspiegelung statt bei einem dotierten Leuchtstoffbereich 19 auch bei einem beschichteten Leuchtstoffbereich 19' Verwendung finden. Außerdem kann eine flexible Lichtleitfaser statt des Glasfaserstabes als Lichtleiter vorhanden sein.

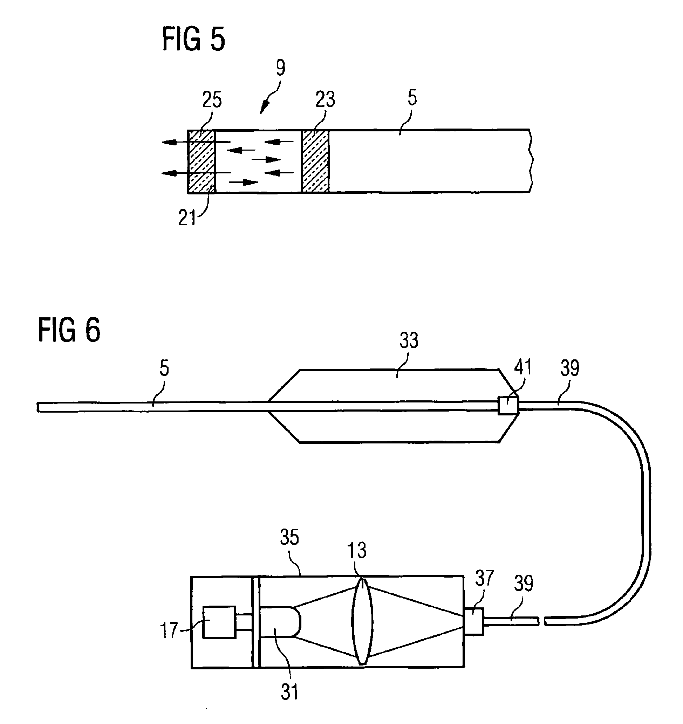

Figur 5 stellt eine Abwandlung des in Figur 4 dargestellten distalen Endes 9 des Glasfaserstabes 5 dar. In dieser Abwandlung ist eine zweite dichroite Verspiegelung 25 am Austrittsende 21 des Glasfaserstabes 5 vorhanden, die das blaue Licht in Richtung auf das proximale Ende 7 des Glasfaserstabes 5 reflektiert, zumindest jedoch dessen phototoxische Wellenlängenbestandteile. Zudem kann die dem Austrittsende 21 mit Abstand in Richtung auf das proximale Ende 7 vorgelagerte dirchroite Verspiegelung 23 in dieser Abwandlung so ausgestaltet sein, dass sie nicht nur das im Leuchtstoffbereich 19 entstehende gelbe Licht reflektiert sondern auch sich vom Austrittsende 21 in Richtung auf das proximale Ende 7 des Glasfaserstabes 5 ausbreitendes blaues Licht. Dieses wird dann so lange zwischen den Spiegeln 23, 25 hin und her reflektiert, bis es vom Konverter-Leuchtstoff vollständig konvertiert ist. In diesem Fall emittiert die sekundäre Lichtquelle des Endoilluminators gelbes Licht mit einer breiten spektralen Verteilung statt weißem Licht.

Eine Abwandlung des mit Bezug auf Figur 1 beschriebenen Endoilluminators ist in Figur 6 dargestellt. Um Wiederholungen zu vermeiden, sind Elemente, die Elementen aus Figur 1 entsprechen, mit denselben Bezugsziffern wie in Figur 1 bezeichnet und werden nicht noch einmal beschrieben.

Im Unterschied zur in Figur 1 dargestellten Ausführungsvariante des Endoilluminators ist der Glasfaserstab 5 in der in Figur 6 gezeigten Variante lediglich von einem einfachen Griffstück 33 umgeben, durch das sich der Glasfaserstab 5 hindurch erstreckt. Die Laserdiode 31, die Einkopplungsvorrichtung 13 sowie die Elektronik 17 sind in einer separaten Versorgungseinheit 35 angeordnet. Diese ist mit einer Aufnahme für einen optischen Steckverbinder 37 ausgestattet, in den ein entsprechender optischer Steckverbinder am proximalen Ende einer Lichtleitfaser 39 eingesteckt werden kann. Das distale Ende dieser Lichtleitfaser 39 kann in einen optischen Steckverbinder 41 am Handgriff 33 eingesteckt werden. Der optische Steckverbinder 41 ist zum Übertragen des aus der Lichtleitfaser 39 austretenden Lichtes in den sich durch das Griffstück 33 erstreckenden Glasfaserstab 5 ausgebildet.

Als Lichtquelle kommt im vorliegenden Ausführungsbeispiel ein Festkörperlaser 31 zur Anwendung, der blaues oder violettes Licht mit einer Wellenlänge unter 420 µm aussendet. Die Ausgestaltung gemäß Figur 6 ermöglicht es, das Griffstück 33 und die mittels des Griffstückes 33 zu handhabenden Elemente des Endoilluminators besonders leicht auszubilden. Außerdem können so auch Elektroniken, Einkopplungsvorrichtungen oder Laser Verwendung finden, die zur Integration in den Handgriff zu sperrig oder schwer wären. Schließlich ermöglicht es diese Ausgestaltung auch, mehrere Lichtleitfasern 39 an eine gemeinsame Versorgungseinheit 35 anzukoppeln, so dass mehrere Glasfaserstäbe 5 von derselben Vorsorgungseinheit 35 mit Laserstrahlung eines leistungsstarken Lasers versorgt werden können.

Das distale Ende des Glasfaserstabs 5 entspricht dem distalen Ende des mit Bezug auf die Figuren 1 bis 5 beschriebenen Endoilluminators. Alternativ ist es jedoch auch möglich, den Leuchtstoffbereich in die Lichtleitfaser 39 zwischen Versorgungseinheit 35 und Handgriff zu verlegen. In diesem Fall bildet nicht das Austrittsende des Glasfaserstabs 5, sondern das Austrittsende der Lichtleitfaser 39 die erfindungsgemäße sekundäre Lichtquelle.

Sowohl der Glasfaserstab 5 als auch die Lichtleitfaser 39 können insbesondere als Monomode-Fasern ausgebildet sein.

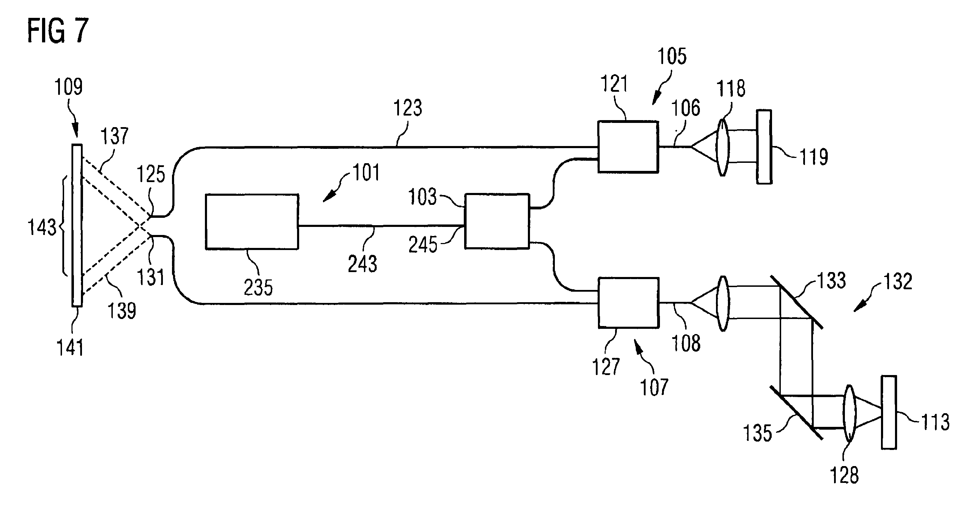

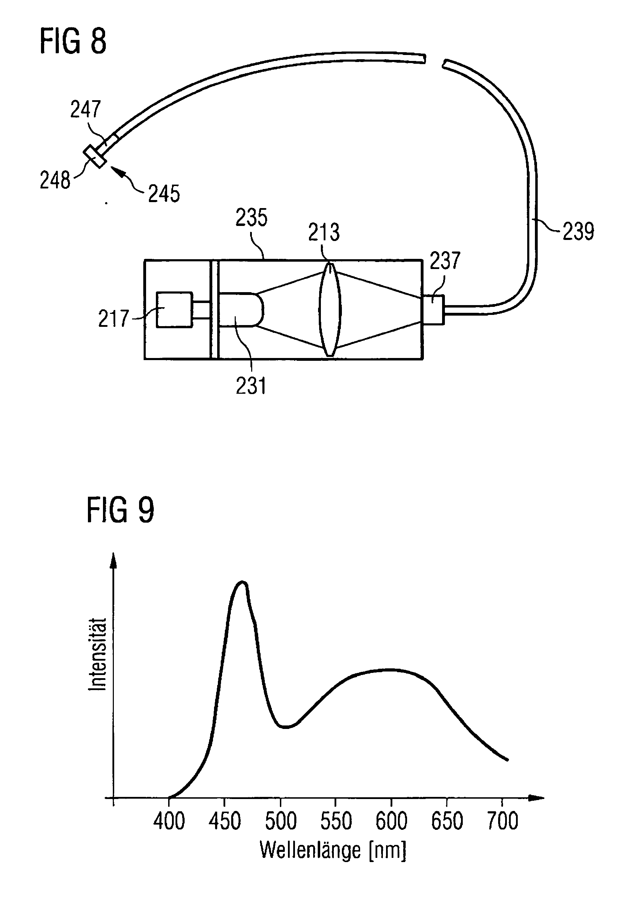

Nachfolgend wird eine OCT-Vorrichtung mit einer erfindungsgemäßen sekundären Lichtquelle mit Bezug auf die Figuren 7 und 8 beschrieben.

Die OCT-Vorrichtung umfasst eine erfindungsgemäße sekundäre Lichtquelle 101 zum Abgeben von zeitlich inkohärentem Licht, einen Aufteiler 103 zum Aufteilen des Lichtes in einen Referenzstrahl und einen Messstrahl, einen Referenzzweig 105, in den der Referenzstrahl vom Aufteiler 103 eingekoppelt wird und in dem dieser einen definierten Weg zurücklegt, einen Messzweig 107, in den der Messstrahl vom Aufteiler 103 eingekoppelt wird und über den der Messstrahl einer Probe 113 zugeführt wird, sowie einen Detektor 109, in dem von der Probe 113 reflektiertes Messlicht mit Referenzlicht aus dem Referenzzweig 105 überlagert und das überlagerte Licht detektiert wird.

Die sekundäre Lichtquelle 101 ist eine Breitbandlichtquelle, die im Wesentlichen zeitlich inkohärente Strahlung abgibt. Sie ist in Figur 8 im Detail dargestellt und umfasst eine Versorgungseinheit 235, die mit der Versorgungseinheit 35 aus Figur 6 übereinstimmt und daher nicht noch einmal im Detail beschrieben wird. An den optischen Steckverbinder 237 der Versorgungseinheit 235 ist eine Lichtleitfaser 243 angeschlossen, deren distales Ende 245 mit einem Leuchtstoffbereich 247 versehen ist, wie er mit Bezug auf Figur 2 beschrieben worden ist. Außerdem weist das distale Ende 245 der Lichtleitfaser 243 einen optischen Steckverbinder 248 auf, der in den Mischer 103 einsteckbar ist.

Selbstverständlich kann das distale Ende 245 der Lichtleitfaser 243 auch mit der mit Bezug auf Figur 4 oder Figur 5 beschriebenen dichroiten Verspiegelung versehen sein. Ebenso ist es möglich, den Leuchtstoffbereich 247 am distalen Ende 245 statt durch Dotierung durch eine Beschichtung zu bilden. Auch der beschichtete Leuchtstoffbereich kann mit einer sich in Richtung auf das proximale Ende der Lichtleitfaser 243 anschließenden dichroiten Verspiegelung versehen sein.

Eine spektrale Verteilung des Lichtes der sekundären Lichtquelle 101, also des aus dem distalen Ende 245 der Lichtleitfaser 243 austretenden Lichtes, bei Verwendung von YAG:Ce als Konverter-Leuchtstoff ist in Figur 9 dargestellt. Die Verteilung weist eine relativ schmale Linie im blauen Spektralbereich mit einer zentralen Wellenlänge von ca. 460 µm und eine breite Verteilung mit einer zentralen Wellenlänge bei ca. 550 µm und einer Breite von ca. 100 µm auf. Die blaue Linie repräsentiert hierbei das nicht konvertierte blaue Licht der Laserdiode 231. Damit ist das von der sekundären Lichtquelle 101 emittierte weiße Licht breitbandig genug für die Verwendung in einer OCT-Vorrichtung. Die Kohärenzlänge der Lichtquelle 101 bestimmt die Tiefenauflösung des OCT. Die Kohärenzlänge des Laserlichtes kann durch gepulsten Betrieb des Lasers verringert werden. Diese spektrale Verteilung liegt auch bei dem mit Bezug auf die Figuren 1 bis 5 beschriebenen Ausführungsbeispiel für einen Endoilluminator mit einer erfindungsgemäßen sekundären Lichtquelle vor. In der mit Bezug auf Figur 5 beschriebenen Abwandlung fehlt jedoch die blaue Linie.

Im Referenzzweig 105 wird der Referenzlichtstrahl in einen Referenzlichtleiter 106 eingekoppelt und über eine Optik 118 einem Spiegel 119 zugeführt. Der Spiegel 119 reflektiert den Referenzstrahl, welcher nach der Reflexion von der Optik 118 wieder in den Referenzlichtleiter 106 eingekoppelt wird. Ein Mischer 121 mischt das reflektierte Referenzlicht mit dem vom Aufteiler 103 kommenden Referenzstrahl im Verhältnis 50:50 und koppelt das derart aufbereitete Licht in einen weiteren, zum Detektor 109 führenden Referenzlichtleiter 123 ein, welcher den Referenzlichtstrahl einem Strahlenausgang 125 des Referenzzweiges 105 zuleitet. Die Referenzlichtleiter und alle anderen Lichtleiter sind vorzugsweise Monomodefasern.

Das Messlicht wird über ein im Messzweig 107 angeordneten Messlichtleiter 108 einer Scaneinrichtung 132 zugeführt, von der er auf eine den Messlichtstrahl auf einen Probenbereich fokussierende Optik 128 gerichtet wird. Die Scaneinrichtung 132 umfasst einen ersten um eine Achse schwenkbaren Galvanometerspiegel 133 zum Vermitteln einer X-Ablenkung des Messstrahls sowie einen zweiten um eine Achse schwenkbaren Galvanometerspiegel 135 zum Vermitteln einer Y-Ablenkung des Messstrahls. Die Achsen, um welche die jeweiligen Galvanometerspiegel 133, 135 schwenkbar sind, stehen vorzugsweise senkrecht zueinander, können jedoch auch beliebige Winkel zueinander einnehmen, solange sie nicht parallel zueinander sind. Mittels einer Scansteuerung (nicht dargestellt) werden die Galvanometerspiegel 133, 135 so gesteuert, dass Schritt für Schritt ein bestimmter Probenbereich gescannt wird. In jedem Scanschritt wird dabei das von der Probe 113 reflektierte Licht von der Mikroskopoptik 128 aufgenommen und über die Scaneinrichtung 132 dem Messlichtleiter 108 wieder zugeführt. Andere als die beschriebene Scanvorrichtung können alternativ zur Anwendung kommen.

Ein Mischer 127, dem das Messlicht über den Messlichtleiter 108 zugeleitet wird, mischt das von der Probe reflektierte Messlicht im Verhältnis 50:50. Das derart aufbereitete Messlicht wird vom Mischer 127 in einen weiteren Messlichtleiter 129, ebenfalls vorzugsweise eine Monomodefaser, einkoppelt, der das Messlicht dem Strahlausgang 131 des Messzweiges 107 zuleitet.

Von den Strahlenausgängen 125, 131 des Referenzzweiges 105 bzw. des Messzweiges 107 wird das Referenzlicht und das Messlicht in Form von Lichtkegeln 137, 139 auf eine CCD-Zeile 141 des Detektors 109 gerichtet, welche die Sensorfläche des Detektors 109 darstellt. Die beiden Strahlenausgänge 125, 131 sind voneinander beabstandet angeordnet, so dass sich die beiden Lichtkegel teilweise überlagern und mindestens einen Teilbereich 143 der CCD-Zeile 141 gleichzeitig beleuchten. Nur, wenn das an einem Punkt der CCD-Zeile 141 ankommende Messlicht die gleiche Wegstrecke zurückgelegt hat wie das am selben Punkt der CCD-Zeile 141 ankommende Referenzlicht, treten Interferenzerscheinungen auf. Aus den bekannten Weglängen, welche das Referenzlicht vom Strahlenausgang 125 bis zu den jeweiligen Punkten auf der CCD-Zeile zurückzulegen hat, lässt sich dem jeweiligen Punkt auf der CCD-Zeile 141 eine Tiefe innerhalb der Probe 113 zuordnen. Nur Messlicht, welches in dieser Tiefe reflektiert worden ist, interferiert an dem zugeordneten Punkt der CCD-Zeile 141 mit dem Referenzlicht. Eine nicht dargestellte Ausleseeinheit liest die CCD-Zeile aus und gibt die ausgelesenen Daten an eine Auswerteeinheit (ebenfalls nicht dargestellt) weiter, welche die Zuordnung eines Pixels zur Probentiefe, aus welcher das auf das Pixel auftreffende Messlicht stammt, vornimmt.

### Bezugszeichenliste

- 1: Endoilluminator
- 3: Handgriff
- 5: Glasfaserstab
- 7: proximales Ende
- 9: distales Ende
- 11: Laserdiode
- 13: Linse
- 15: optischer Steckverbinder
- 17: Elektronik
- 19: Leuchtstoffbereich
- 19': Leuchtstoffbereich
- 21: Austrittsende
- 23: Verspiegelung
- 31: Laser
- 33: Griff
- 35: Versorgungseinheit
- 37: optischer Steckverbinder
- 39: Lichtleitfaser
- 41: optischer Steckverbinder
- 43: Lichtleitfaser
- 45: distales Ende
- 47: Leuchtstoffbereich
- 101: sekundäre Lichtquelle
- 103: Aufteiler
- 105: Referenzzweig
- 106: Lichtleitfaser
- 107: Messzweig
- 108: Lichtleitfaser
- 109: Defektor
- 113: Probe
- 118: Optik
- 119: Spiegel
- 121: Mischer
- 123: Lichtleitfaser
- 125: Ausgang
- 127: Mischer
- 128: Optik
- 131: Ausgang
- 132: Scaneinrichtung
- 133: Spiegel
- 135: Spiegel
- 137: Lichtkegel
- 139: Lichtkegel
- 141: CCD-Zeile
- 143: Teilbereich
- 235: Versorgungseinheit
- 237: optischer Steckverbinder
- 239: Lichtleitfaser
- 241: optischer Steckverbinder
- 243: Lichtleitfaser
- 245: distales Ende
- 247: Leuchtstoffbereich
- 248: optischer Steckverbinder

## Patentansprüche

1. Sekundäre Lichtquelle mit:
- einer schmalbandiges Licht emittierenden schmalbandigen Lichtquelle (11, 31, 231) als einer primären Lichtquelle,
- einem Lichtleiter (5, 243) mit einem proximalen und einem distalen Ende (9, 245)
- einer am proximalen Ende (7) des Lichtleiters (5, 243) angeordneten Einkopplungsvorrichtung (13, 213) zum Einkoppeln des schmalbandigen Lichts in den Lichtleiter (5, 243), und
- einem an oder vor dem distalen Ende (9, 245) des Lichtleiters (5, 243) vorhandenen Leuchtstoffbereich (19, 19', 247), der mit einem Konverter-Leuchtstoff versehen ist,
wobei der Konverter-Leuchtstoff des Leuchtstoffbereiches (19, 19', 247) derart im Bezug auf das von der schmalbandigen Lichtquelle (11, 31, 231) emittierte schmalbandige Licht gewählt ist, dass die Wellenlänge wenigstens eines Teils des schmalbandigen Lichtes von ihm vergrößert wird.

2. Sekundäre Lichtquelle nach Anspruch 1, bei der der Konverter-Leuchtstoff des Leuchtstoffbereiches (19, 19', 247) derart im Bezug auf das von der schmalbandigen Lichtquelle (11, 31, 231) emittierte schmalbandige Licht gewählt ist, dass am distalten Ende (9, 245) des Lichtleiters weißes oder breitbandiges Licht austritt.

3. Sekundäre Lichtquelle nach Anspruch 1 oder 2, bei der der Konverter-Leuchtstoff als Dotierung in den Leuchtstoffbereich (19, 247) des Lichtleiters (5, 243) eingebracht ist.

4. Sekundäre Lichtquelle nach Anspruch 1 oder 2, bei der sich der Leuchtstoffbereich (19') am distalen Ende (9) des Lichtleiters (5) befindet und der Konverter-Leuchtstoff des Leuchtstoffbereiches (19') als Beschichtung auf das distale Ende (9) des Lichtleiters (5) aufgebracht ist.

5. Sekundäre Lichtquelle nach einem der Ansprüche 1 bis 4, bei der sich eine dichroite Verspiegelung (23) in Richtung auf das proximale Ende (7) des Lichtleiters an den Leuchtstoffbereich (19) anschließt.

6. Sekundäre Lichtquelle nach einem der Ansprüche 1 bis 5, bei der am Austrittsende (21) des Lichtleiters eine dichroite Verspiegelung (25) vorhanden ist.

7. Sekundäre Lichtquelle nach einem der Ansprüche 1 bis 6, bei der der Lichtleiter ein Glasfaserstab (5) ist.

8. Sekundäre Lichtquelle nach einem der Ansprüche 1 bis 7, bei der der Lichtleiter (5, 243) eine Monomode-Faser ist.

9. Sekundäre Lichtquelle nach einem der Ansprüche 1 bis 8, bei schmalbandige Lichtquelle (11, 31, 231) blaues Licht emittiert und der Konverter-Leuchtstoff derart gewählt ist, dass das blaue Licht in weißes Licht oder breitbandiges Licht umgewandelt wird.

10. Sekundäre Lichtquelle nach einem der Ansprüche 1 bis 9, bei der der Konverter-Leuchtstoff ausgewählt ist aus der Gruppe YAG:Ce, ThAG:Ce, SrGa₂S₄:Eu, Ca₈EuMnMg(SiO₄)₄C₁₂, CaS:Eu, SrS:Eu oder Gemischen daraus.

11. Sekundäre Lichtquelle nach einem der Ansprüche 1 bis 10, bei der die schmalbandige Lichtquelle eine Diode (11) ist.

12. Sekundäre Lichtquelle nach einem der Ansprüche 1 bis 11, bei der die schmalbandige Lichtquelle ein Laser (11) ist.

13. Medizinische Illuminationsvorrichtung mit einer sekundären Lichtquelle nach einem der Ansprüche 1 bis 12.

14. Medizinische Illuminationsvorrichtung nach Anspruch 13, die als Endoilluminator (1) ausgestaltet ist.

15. Optisches Kohärenzmikroskop mit einer sekundären Lichtquelle nach einem der Ansprüche 1 bis 12
